# EUROPÄISCHE PATENTSCHRIFT

(11) **EP 0 811 398 B1**
(45) Veröffentlichungstag und Bekanntmachung des Hinweises auf die Patenterteilung: **23.10.2002**
(21) Anmeldenummer: 97250169.6
(22) Anmeldetag: 02.06.1997
(51) Int. Cl.: A61N 1/378, A61N 1/37

(54) **Implantierbares Stimulationsgerät**
Implantable pacemaker
Stimulateur implantable

(30) Priorität: 04.06.1996 DE 19623788
(43) Veröffentlichungstag der Anmeldung: 10.12.1997
(73) Patentinhaber: BIOTRONIK Mess- und Therapiegeräte GmbH & Co Ingenieurbüro Berlin, 12359 Berlin (DE)
(72) Erfinder: Braun, Hans-Jürgen, Dipl.-Ing., 12307 Berlin (DE)
(74) Vertreter: Christiansen, Henning

(56) Entgegenhaltungen:
- EP-A- 0 431 437
- US-A- 4 481 950
- US-A- 4 548 209
- US-A- 5 387 228
- US-A- 5 488 553

## Beschreibung

Die Erfindung betrifft ein implantierbares Stimulationsgerät der im Oberbegriff des Anspruchs 1 angegebenen Art.

Implantierbare Stimulationsgeräte - von denen Herzschrittmacher die größte Bedeutung erlangt haben, unter denen aber auch implantierbare Defibrillatoren sowie sonstige Muskel-, Nerven- oder Knochenstimulatoren für den Langzeiteinsatz zunehmende Verbreitung finden - haben sich in den letzten Jahren, beginnend mit der Einführung des Mikroprozessors Ende der siebziger Jahre, zunehmend zu Geräten mit großer Funktionsvielfalt und umfangreichen internen Erkennungsund Anpassungsfunktionen entwickelt. Dies ist mit zunehmender Komplexität und Bedeutung ihrer Steuerung für die sichere Gerätefunktion einhergegangen, so daß die Gewährleistung einer ununterbrochenene Versorgung der digitalen Steuerschaltungen mit ausreichender Betriebsspannung eine elementare Anforderung an alle modernen derartigen Geräte ist; vgl. dazu etwa US 4 448 197.

Die genannten Geräte sind üblicherweise batteriebetrieben. Als Batterien werden heute praktisch ausschließlich Lithiumbatterien eingesetzt, die eine sowohl für die Stromversorgung der Steuerschaltung(en) auf Basis integrierter Schaltkreise - speziell in CMOS-Technik - als auch für viele Stimulationsaufgaben ausreichende Spannung von ca. 3 V liefern und eine hohe spezifische Energiespeicherkapazität aufweisen; vgl. W. Greatbatch: "A New Pacemaking System Utilizing a Long-life Lithium Cell; Dig. 9th Intern. Conf. on Medical and Biological Eng., Melbourne 1971.

Wie jede elektrochemische Zelle haben aber auch Lithiumbatterien eine Entladungskennlinie, die durch einen Anstieg des Klemmwiderstandes mit zunehmender Entladung, d.h. zunehmender Einsatzdauer des implantierten Gerätes, geprägt ist. Die Aufrechterhaltung einer sicheren Gerätefunktion erfordert jedoch die Abgabe von Stimulationsimpulsen mit definierter, konstanter oder in vielen Einsatzfällen (etwa von Herzschrittmachern) sogar nach einer gewissen Einsatzdauer auf höhere als die Anfangswerte einstellbarer, Spannung. Zudem gibt es - wiederum speziell bei Herzschrittmachern, insbesondere aber auch bei Defibrillatoren bzw, Kardiovertern - auch Einsatzfälle, in denen die Klemmenspannung auch einer neuen Lithiumzelle für eine (sichere) Erfüllung der Stimulationsaufgabe nicht ausreicht.

Aus diesen Gründen weisen Herzschrittmacher - wie bereits in der obengenannten Veröffentlichung von GREATBATCH und detaillierter etwa in der US-Patentschrift 3 707 974 vorgeschlagen Ausgangsschaltungen mit Mitteln zur Erhöhung (speziell Vervielfachung) der Ausgangsspannung gegenüber der Zellen-Klemmenspannung auf. Diese Mittel werden vielfach als "Ladungspumpe" oder - nach den Haupt-Funktionselementen, nämlich geschalteten Kondensatoren - als SC (switched-capacitor)-Transformer bezeichnet und umfassen üblicherweise einen sogenannten Pumpkondensator, über den Energie aus der Batterie in die Ausgangsstufe übertragen wird, und diesem zugeordnete Schaltmittel zur Steuerung der Energieübertragung. Eine auf digitaler Basis arbeitende, extern programmierbare Ladungspumpe mit mehreren Pumpkondensatoren und Schaltern zur Realisierung verschiedener Ausgangsspannungen ist in der europäischen Patentschrift EP 0 000 983 B1 beschrieben. Eine moderne Anordnung dieser Art, mit der in Abängigkeit vom Erschöpfungsgrad der Batterie geregelte oder ungeregelte Stimulationsspannungen bereitgestellt werden können, ist in der US-Patentschrift 5 387 228 beschrieben.

Es ist auch bekannt, Herzschrittmacher mit Mitteln zur Anzeige einer weitgehenden Erschöpfung der Batterie - einer sogenannten EOL(end-of-life)-Detektion, auszurüsten, um eine rechtzeitige Implantation eines neuen Schrittmachers zu sichern, bevor lebenbedrohliche Funktionsausfälle auftreten.

Aus EP 0 463 410 B1 ist ein implantierbares Stimulationsgerät bekannt, bei dem für die Zeitsteuerung des Impulsgenerators in Abhängigkeit vom Erschöpfungszustand der Batterie ein kleinster zulässiger Wert des Stimulationsintervalls vorgegeben wird.

In EP 0 431 437 A2 wird die Umschaltung auf eine niedrigere als die jeweils über einen physiologischen Sensor indizierte Stimulationsrate oder aus einer Betriebsart mit hohem Stromverbrauch in eine solche mit niedrigerem Stromverbrauch bei Unterschreitung einer vorgegebenen Batteriespannungsschwelle vorgeschlagen.

In der US 4 548 209 wird ein implantierbarer Kardioverter beschrieben, bei dem das Zeitregime bei der Aufladung des Hochspannungskondensators zur Bereitstellung der Kardioversionsspannung (DC-DC-Wandlung) über induktive Kopplung in Abhängigkeit von der Spannung bzw. vom Innenwiderstand der Batterie gesteuert wird.

Bei den beschriebenen Ladungspumpen tritt das Problem auf, daß ihre Aktivierung ohne Beachtung des Erschöpfungsgrades der Batterie zu einem unkontrollierten Absinken der Klemmenspannung führt, womit die Gefahr besteht, daß diese die für den Betrieb der Steuerschaltungen erforderliche Mindestspannung unterschreitet. Das kann äußerst nachteilige, ggfs. irreparable, Folgen für die Funktion des Stimulationsgerätes haben. Das genannte Problem kann insbesondere dann praktisch relevant werden, wenn im Ergebnis einer Reizschwellenmessung nach bereits längerer Betriebsdauer eines Schrittmachers (d.h. bei bereits weitgehend entladener Batterie) eine erhöhte Stimulationsamplitude programmiert werden muß.

Einen Ansatz, das beschriebene Problem zu lösen, ist in US 5 488 553, in des ein implantierbares Stimulationsgerat gemäß dem Oberbegriff von Anspruch 1 beschrieben ist, offenbart.

Gegenüber diesem Stand der Technik stellt sich die Aufgabe, das offenbarte Stimulationsgerät weiter zu verbessern.

Diese Aufgabe wird durch ein Stimulationsgerät mit den Merkmalen des Anspruchs 1 gelöst.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen
Figur 1 ein stark vereinfachtes Funktions-Blockschaltbild der für die Ausführung der Erfindung wesentlichen Teile eines Stimulationsgerätes gemäß einer Ausführungsform,
Figur 2 ein Funktions-Blockschaltbild einer modifizierten Ausführungsform und
Figur 3 eine Detaildarstellung zur Anordnung gemäß Fig. 2.

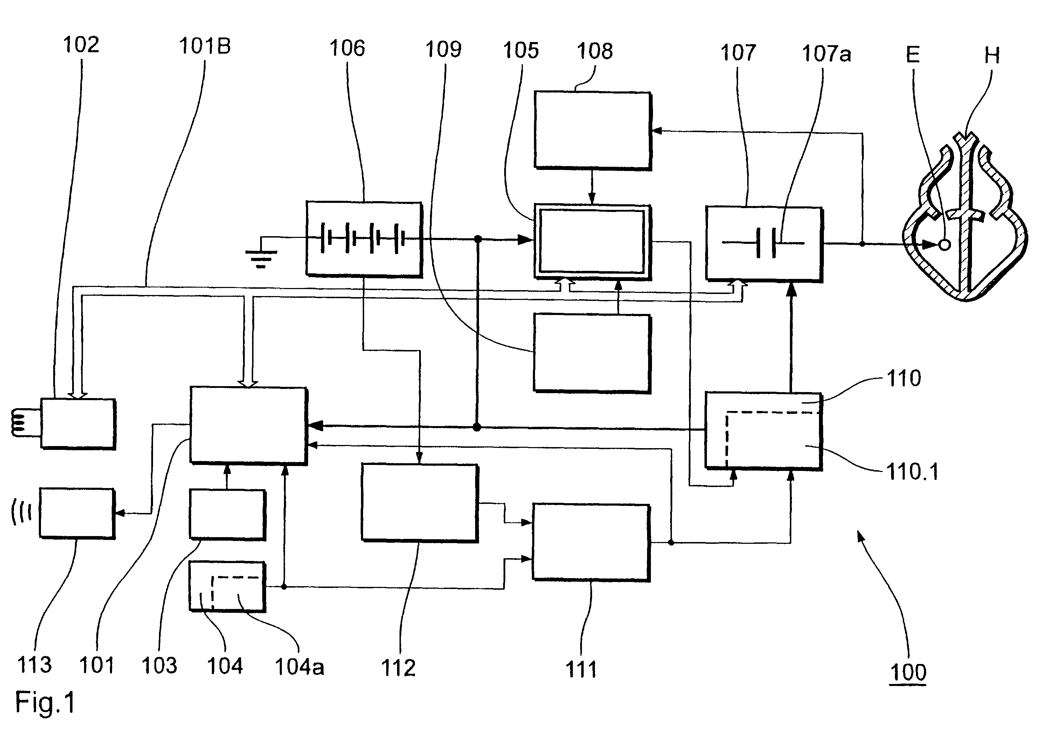

Figur 1 zeigt schematisch wesentliche Komponenten eines Einkammer-Bedarfsschrittmachers 100, der über eine Elektrode E zum einen zum Abfühlen von natürlichen Herzaktionen und zum anderen zur bedarfsweisen (intermittierenden) Abgabe von Stimulationsimpulsen mit der rechten Herzkammer eines Herzens H verbunden ist. Als wesentliche Steuerbaugruppen des Schrittmachers 1 sind ein Mikroprozessor 101, dem in üblicher Weise eine Telemetrieeinheit 102 sowie ein Programmspeicher 103 und ein Datenspeicher 104 zugeordnet sind, sowie eine Controller-/Zeitsteuereinheit 105 gezeigt.

Mit stark durchgezogenen Linien ist symbolisiert, daß diese Steuerbaugruppen ebenso mit einer Lithiumbatterie 106 zur Stromversorgung verbunden sind wie (indirekt über einen weiter unten spezifizierten Block) eine Ausgangsstufe 107; alle weiteren Stromversorgungsleitungen sind der besseren Übersichtlichkeit der Darstellung halber weggelassen. Die Ausgangsstufe umfaßt - wie durch den mit 107a bezeichneten Kondensator symbolisiert - eine Speicherkondensatoranordnung zur Speicherung elektrischer Energie zur Erreichung einer gegenüber der Batteriespannung erhöhten Stimulationsamplitude.

Als Mittel zum Daten- und Steuersignalaustausch wischen dem Mikroprozessor 101, der Telemetrieeinheit 102, der Controller-/Zeitsteuereinheit 105 und der Ausgangsstufe 107 ist ein Daten- und Steuersignalbus 101B dargestellt. Mit der Elektrode E ist in üblicher Weise eine Eingangsstufe 108 verbunden, die in Datensignalverbindung mit einem Steuereingang der Controller-/Zeitsteuereinheit 105 steht. Dieser ist eingangsseitig ferner eine Oszillatorschaltung 109 zugeordnet.

Ausgangsseitig ist die Controller-/Zeitsteuereinheit 105 - neben den über die Datenbus 101B realisierten Verbindungen - über eine Steuersignalleitung mit einem von zwei Steuereingängen einer Pumpsteuerschaltung 110 verbunden, die ihrerseits in die Stromversorgungsleitung zwischen der Batterie 106 und der Ausgangsstufe 107 eingeschleift ist. Die Pumpsteuerschaltung 110 weist einen zweiten Steuereingang auf, über den sie mit dem Ausgang einer Vergleichereinheit 111 verbunden ist, deren einer Eingang mit einem Batteriespannungsfühler 112 und deren anderer Eingang mit einem Betriebsspannungs-Speicherbereich 104a des Datenspeichers 104 verbunden ist. Der Ausgang der Vergleichereinheit 111 ist weiter mit einem Eingang des Mikroprozessors 101 verbunden, und diesem wiederum ist ein Warnsignalgeber 113 zugeordnet.

Die Stimulationimpulserzeugung und -abgabe und die meisten weiteren Betriebsabläufe werden bei dieser Anordnung in an sich bekannter Weise gesteuert, so daß diesbezüglich keine spezielle Erläuterung erforderlich ist.

Im Hinblick auf die Ausführung der Erfindung wesentlich ist zunächst die aus dem Batteriespannungsfühler 112, dem Betriebspannung-Speicherbereich 104a und der mit diesen verbundenen Vergleichereinheit 111 gebildete Baugruppe zur Erfassung der Relation zwischen der aktuellen Batteriespannung und einer gespeicherten Mindest-Betriebsspannung des Mikroprozessors 101 und der Controller-/Zeitsteuereinheit 105 (resp. weiterer Baugruppen des Schrittmachers). Wesentlich ist ferner, daß die mittels der Vergleichereinheit 111 festgestellte Relation zur Steuerung der Energieübertragung von der Batterie 106 zur Ausgangsstufe 107 genutzt wird.

Diese läuft im einfachsten Fall so ab, daß das von der Vergleichereinheit 111 im Falle einer Unterschreitung der Mindestspannung durch die aktuelle Batteriespannung ausgegebene Signal an die Pumpsteuerschaltung 110 als Ansteuersignal für ein in dieser vorgesehenes strombegrenzendes Bauelement (CMOS-Transistor, FET o.ä.) bzw. eine entsprechende Schaltung aus solchen an dessen bzw. deren Steueranschluß angelegt wird. Nach Ausgabe dieses Steuersignals durch die Vergleichereinheit 111 wird somit der maximale Effektivwert des Pumpstroms aktiv begrenzt, d.h. gegenüber dem vorher eingestellten, üblicherweise widerstandsbegrenzten Wert reduziert. Hierdurch wird die Belastung der Batterie verringert, woraufhin sich die Batteriespannung wieder etwas erhöhen wird. Im Ergebnis wird sich hierbei ein für den aktuellen Batterieentladungszustand höchstzulässiger mittlerer Pumpstrom einstellen.

Da im Normalbetrieb eines Schrittmachers die Ladungspumpschaltung nur während eines Teils der Zeitspanne zwischen den Stimulationsimpulsen arbeitet, kann eine Verringerung des Pumpstroms in bevorzugter Weise durch eine Verlängerung der Pumpdauer für den Transport einer vorbestimmten Ladungsmenge in die Ausgangsstufe (innerhalb der durch das gültige Stimulationsimpulsintervall gesetzten Grenzen) ausgeglichen werden; siehe dazu weiter unten. Die programmierte Stimulationsamplitude wird daher trotz der Pumpstrombegrenzung noch für eine bestimmte Restbetriebsdauer des Schrittmachers erreicht werden.

Zur Sicherheit wird dennoch das eine Pumpstrombegrenzung in der Pumpsteuerschaltung 110 bewirkende Ausgangssignal der Vergleichereinheit 111 zugleich dem Mikroprozessor 101 zugeführt. Dieser leitet hieraus - ggfs. nach einer zusätzlich korrigierenden Signalverarbeitung - ein Ansteuersignal für den Warnsignalgeber 113 ab, der (etwa über ein akustisches Signal, einen zusätzlichen elektrisches Reizimpuls o.ä.) unmittelbar dem Patienten das Vorliegen einer Pumpstrombegrenzung und damit die Notwendigkeit einer umgehenden Konsultation des Arztes signalisiert.

Tritt die Pumpstrombegrenzung als Folge dessen auf, daß der Arzt während einer Nachsorgeuntersuchung im Ergebnis eines Reizschwellentests über die Telemetrieeinheit 102 eine erhöhte Stimulationsamplitude zu programmieren versucht, so wird er bereits unmittelbar nach dem Programmierungsvorgang über diese Folge informiert und kann aufgrund des Warnsignals - oder eines zusätzlichen, über die Telemetrieeinheit übertragenen Rückmeldungssignals des Mikroprozessors 101 - die Umprogrammierung abbrechen und/oder Festlegungen hinsichtlich der Implantation eines neuen Schrittmachers treffen.

Die Erfindung beschränkt sich in ihrer Ausführung nicht auf das vorstehend angegebene bevorzugte Ausführungsbeispiel. Vielmehr ist eine Vielzahl von Varianten denkbar, welche von der dargestellten Lösung auch bei anderer Struktur des Blockschaltbildes und Einsatz anderer Bauelemente bzw. Baugruppen Gebrauch machen.

Die Sicherheit für den Patienten kann beispielsweise dadurch weiter erhöht werden, daß anstelle eines einzelnen Wertes einer minimalen Betriebsspannung mehrere betragsmäßig gestaffelte Sicherheits-Spannungswerte gespeichert werden und die Vergleichereinheit als mehrstufiger Schwellwertdiskriminator ausgebildet wird, so daß sie unterschiedliche Ausgangssignale in Abhängigkeit von der Unterschreitung jeweils eines bestimmten der gespeicherten Betriebsspannungs-Schwellwerte ausgibt, eine als "Tabellarischer EOL(End-of-Life)" zu bezeichnende Funktion.

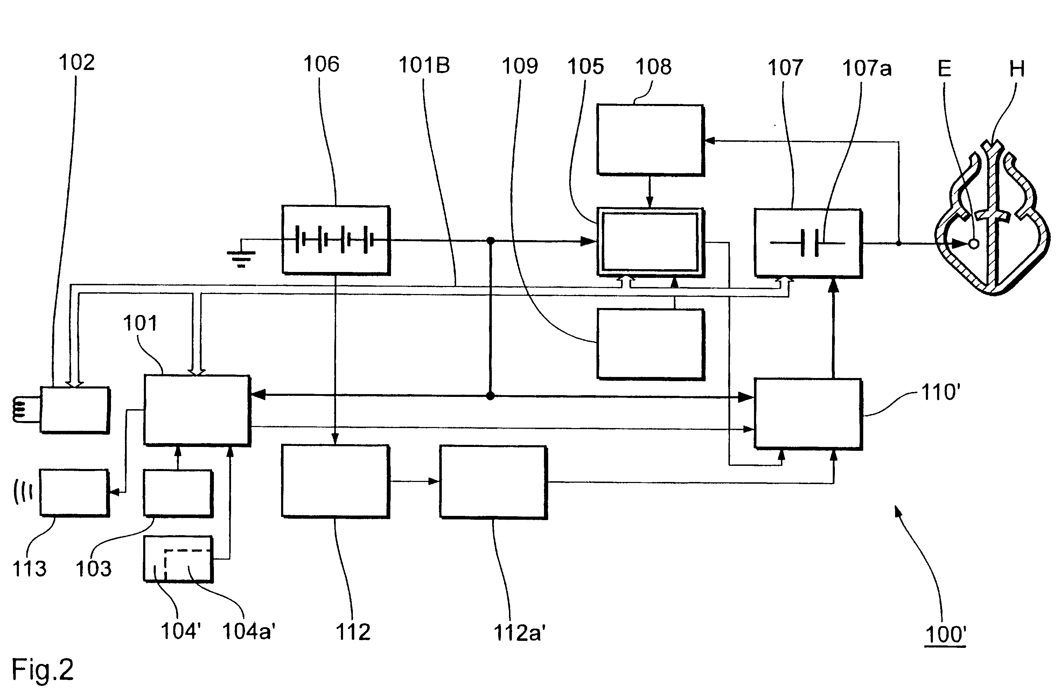

Diese Funktion kann auch mit der in Fig. 2 dargestellten modifizierten Ausführung eines Schrittmachers realisiert werden. Der Schrittmacher 100' nach Fig. 2 unterscheidet sich von der oben unter Bezugnahme auf Fig. 1 beschriebenen Ausführung durch einen dem Batteriespannungsfühler 112 nachgeschalteten A/D-Wandler 112a' und eine wesentlich modifizierte Pumpsteuerschaltung 110'. Im übrigen ist hier auf einen Warnsignalgeber verzichtet, und der Speicher 104 ist daher nur mit dem Mikroprozessor 101 verbunden.

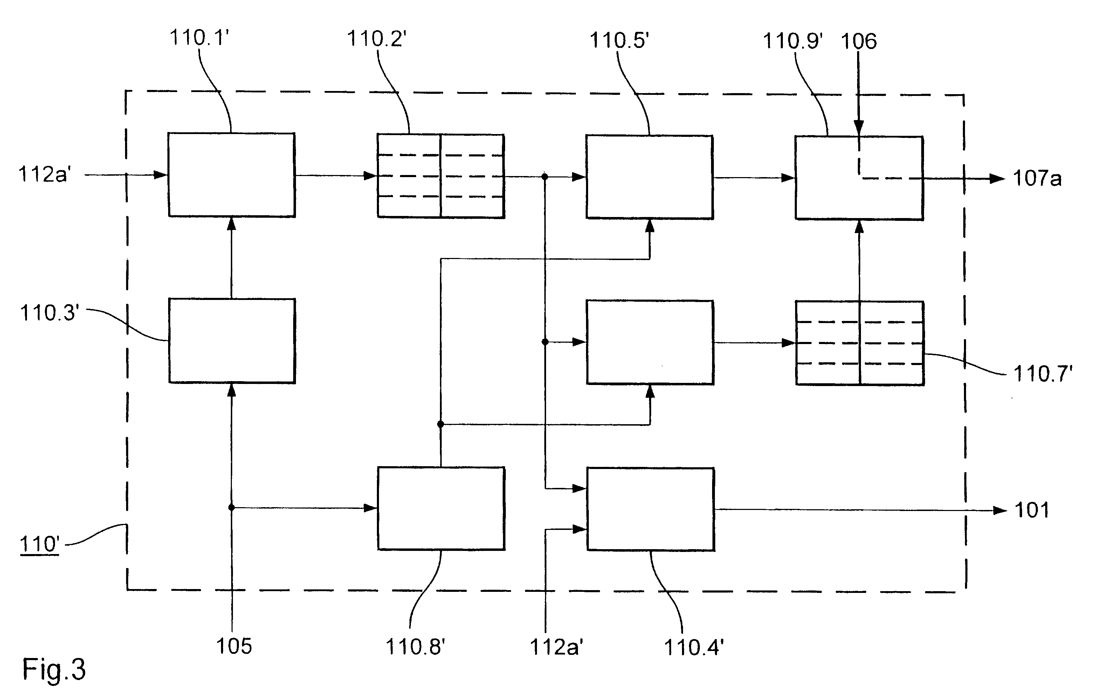

Eine vorteilhafte Ausführung der Pumpsteuerschaltung 110' aus Fig. 2 ist in Fig. 3 skizziert. Sie umfaßt demnach eine mit dem Ausgang des A/D-Wandlers 112a' verbundene Speicherzugriffssteuerung 110.1' zur Adressierung eines Speicherbereiches eines als Lookup-Table aufgebauten Speichers 110.2'. in dem für einen Wert der Stimulationsamplitude bzw. -energie vorgegebene Wertepaare aus Batteriespannungswerten und Pumpzeitintervallwerten gespeichert sind, entsprechend der aktuellen Batteriespannung. Die gespeicherten Wertepaare sind so vorgewählt, daß abnehmenden Batteriespannungswerten zunehmende Pumpzeitintervalle zugeordnet sind, bei deren Anwendung die Batterielast während des Pumpvorganges sukzessive verringert ist. Der niedrigste Spannungswert der im Speicher 110.2' gespeicherten Tabelle entspricht der im Speicher 104a gespeicherten Minimalspannung für den Betrieb der Schrittmachersteuerung. Bei einer Umprogrammierung der Stimulationsenergie ist - je nach Umfang der vorgespeicherten Datenbasis - entweder der Speicher 110.2' insgesamt neu zu programmieren oder der Zugriff auf eine andere Wertetabelle (entsprechend dem neuen Wert der Stimulationsenergie) zu realisieren.

Die Speicherzugriffssteuerung erhält ein Freigabesignal durch einen Zugriffs-Freigabeschalter 110.3', der über den Controller 105 gesteuert wird und einen Speicherzugriff nach Maßgabe des aktuellen Batteriespannungspegels jeweils kurz nach Abgabe eines Stimulationsimpulses ermöglicht. Der aus dem Speicher 110.3' ausgelesene Pumpzeitintervallwert wird als Zähl-Einstellwert einem Zähler 100.5' sowie parallel hierzu über eine weitere Speicherzugriffssteuerung 110.6' einem weiteren Speicher 110.7' zugeführt, in dem - wiederum tabellarisch - Werte einer Steuergröße eines aktiven Pumpstrombegrenzers in Zuordnung zu Werten des Pumpzeitintervalls gespeichert sind.

Der Takteingang des Zählers 110.5' ist über ein Verzögerungsglied 110.8' mit dem Controller 105 verbunden. Der Zähler 110.5' wird nach einer zur Bestimmung des anzuwendenden Pumpzeitintervalls ausreichenden Verzögerung im Verzögerungsglied 110.8' durch den Controller gestartet und zählt von diesem übermittelte Taktimpulse bis zur Erreichung des aktuell eingestellten Pumpzeitintervalls. Beim Start des Zählers gibt dieser einen Einschaltimpuls an einen zwischen die Batterie 106 und den Ausgangskondensator 107a geschalteten Pumpstromschalter/-begrenzer 110.9' ab.

An diesen wird gleichzeitig der aktuelle Wert der Steuergröße aus dem Speicher 110.7' übermittelt. Bei Erreichung des dem eingestellten Pumpzeitintervalls entsprechenden Zählwertes gibt der Zähler 110.5' ein den Pumpstromschalter-/begrenzer wieder sperrendes und die Batterie somit von der Ausgangsstufe trennendes Signal ab, durch das der Pumpvorgang mithin beendet wird.

Der aus dem Speicher 110.2' ausgelesene Pumpzeitintervallwert wird auch dem einen Eingang einer Vergleichereinheit 110.4' zugeführt, an dessen anderem Eingang der vom Mikroprozessor 101 übermittelte aktuelle Wert des Stimulationsimpulsintervalls anliegt. Ist der nach Maßgabe des Batteriespannungswertes einzustellende Pumpzeitintervallwert kleiner als das aktuelle Stimulationsintervall, so wird ohne weiteres die oben skizzierte Funktion ausgeführt.

Ist das Pumpzeitintervall hingegen größer, d.h. ist der Erschöpfungszustand der Batterie so weit fortgeschritten, daß infolge der Ladestrombegrenzung die Zeitspanne des Stimulationsintervalls nicht mehr zum Aufbau der erforderlichen Stimulationsspannung am Ausgangskondensator ausreicht, gibt die Vergleichereinheit ein entsprechendes Steuersignal an den Mikroprozessor 101 aus. Aufgrund eines vorgespeicherten Ablaufes kann auf dieses Signal hin durch Verlängerung des Stimulationsintervalls (Verringerung der Stimulationsrate) und/oder Deaktivierung bestimmter energieintensiver Funktionen der Steuereinrichtung (z.B. der Ratenadaption) reagiert werden, d.h. durch Übergang in einen Notbetriebsmodus. Hierbei kann zudem die oben erwähnte Funktion des tabellarischen EOL zum Tragen kommen, d.h. eine Abstufung des Sicherheitsbetraganteils der Stimulationsenergie erfolgen. Tritt die besagte Situation bei einer Umprogrammierung des Schrittmachers auf, wenn der Arzt die Stimulationsenergie erhöhen will, so löst das (auf übliche Weise über Telemetrie übertragene) Signal eine Warnung auf der Anzeige des Programmiergerätes aus, auf deren Erscheinen hin der Arzt die vorgesehene Programmierung überdenken oder eine Entscheidung zum Austausch des Schrittmachers treffen kann.

Im Rahmen der möglichen Modifikationen der Ausführung der Erfindung kann der in obigen Beispielen eingesetzte Batteriespannungsfühler durch eine Einrichtung zur Erfassung des Batterie-Innenwiderstandes auf dem Wege einer Strommessung ersetzt sein. Weiter kann die Erfassung des tatsächlichen Batteriezustandes durch die Erfassung und geeignete Verarbeitung von Daten zur Betriebs-Vorgeschichte ersetzt sein. Bei einer solchen Ausführung sind Mittel zur Erfassung der effektiven verstrichenen Betriebszeit, Mittel zur Speicherung von Normwerten des Batteriezustandes (z.B. der erwarteten Klemmenspannung in Abhängigkeit von der effektiven Betriebsdauer) sowie entsprechende Vergleichermittel vorzusehen, deren Ausgangssignal an die Stelle eines Batteriezustands-Meßwertes treten kann.

## Patentansprüche

1. Implantierbares Stimulationsgerät (100), mit einer erschöpfbaren Spannungsquelle (106) zur Energieversorgung, einer Steuereinrichtung (101, 105) zur Steuerung eines Stimulationsbetriebes, einer Ausgangsstufe (107) mit einem Stimulationsenergiespeicher (107a), Mitteln zur Erzeugung einer gegenüber der Klemmenspannung der erschöpfbaren Spannungsquelle erhöhten Ausgangsspannung durch Energiezufuhr aus der erschöpfbaren Spannungsquelle in den Stimulationsenergiespeicher und einer Pumpsteuereinrichtung (110, 111, 112; 110', 112a') zur zeitlichen und/oder Stromstärke-Steuerung der Energiezufuhr in den Stimulationsenergiespeicher in Abhängigkeit vom Erschöpfungszustand der Spannungsquelle, wobei die Pumpsteuereinrichtung Mittel (112; 112a') zur Erfassung des Zustandes, insbesondere der Klemmenspannung, der Spannungsquelle (106) und eine mindestens mittelbar eingangsseitig mit den Erfassungsmitteln verbundene steuerbare Pumpstrom-Begrenzerschaltung (110.9') und/oder Pumpzeitintervall-Steuereinrichtung (110.2', 110.5') zur Begrenzung des Pumpstromes in Verbindung mit einer Verlängerung des Pumpzeitintervalls bei zunehmender Erschöpfung der Spannungsquelle aufweist, so daß eine für sicheren Betrieb der Steuereinrichtung erforderliche Mindestbetriebsspannung nicht unterschritten wird, **gekennzeichnet durch** einen über eine erste Speicherzugriffssteuerung (110.1') mit dem Ausgang der Erfassungsmittel (112, 112a') verbundenen ersten Direktzugriffsspeicher (110.2') zur Speicherung einer Mehrzahl von Werten des Pumpzeitintervalls und einen über eine zweite Speicherzugriffsteuerung (110.6') adressierbaren zweiten Direktzugriffsspeicher (110.7') zur Speicherung von Werten einer Steuergröße der Pumpstrom-Begrenzerschaltung (110.9').

2. Implantierbares Stimulationsgerät nach Anspruch 1, **dadurch gekennzeichnet, dass** der erste Direktzugriffsspeicher (110.2') ein programmierbarer Speicher ist.

3. Implantierbares Stimulationsgerät nach Anspruch 1 oder Anspruch 2, **dadurch gekennzeichnet, dass** der zweite Direktzugriffsspeicher (110.7') ein programmierbarer Speicher ist.

4. Implantierbares Stimulationsgerät nach einem der Ansprüche 1 bis 3, **dadurch gekennzeichnet, daß** die Pumpzeitintervall-Steuereinrichtung (110.2', 110.5') zur stufenweisen Verlängerung des Pumpzeitintervalls in den Grenzen des Zeitintervalls zwischen zwei Stimulationsimpulsen im Ansprechen auf ein zunehmende Erschöpfung der Spannungsquelle (106) kennzeichnendes Signal der Erfassungsmittel (112; 112a') hin ausgebildet ist.

5. Implantierbares Stimulationsgerät nach einem der vorangehenden Ansprüche, **gekennzeichnet durch** eine über einen ersten Eingang mit den Erfassungsmitteln (112) und über einen zweiten Eingang mit einem Festwertspeicher (104a) zur Speicherung der Mindestbetriebsspannung sowie ausgangsseitig mit einem Steuereingang der Pumpstrom-Begrenzerschaltung (110) verbundene Vergleichereinheit (111).

6. Implantierbares Stimulationsgerät nach Anspruch 5, **dadurch gekennzeichnet, daß** der Festwertspeicher (104a) zur Speicherung mehrerer abgestufter Betriebspannungs-Schwellwerte und die Vergleichereinheit (111) als mehrstufiger Schwellwertdiskriminator ausgebildet ist derart, daß die Vergleichereinheit unterschiedliche Ausgangssignale in Abhängigkeit von der Unterschreitung jeweils eines bestimmten der gespeicherten Betriebsspannungs-Schwellwerte am Batteriespannungsfühler (112) ausgibt.

7. Implantierbares Stimulationsgerät nach einem der Ansprüche 1 bis 6, **dadurch gekennzeichnet, daß** die Pumpstrom-Begrenzerschaltung (110.9') einen, insbesondere mehrstufigen, Spitzenstrombegrenzer aufweist.

8. Implantierbares Stimulationsgerät nach einem der Ansprüche 1 bis 7, **gekennzeichnet durch** einen mindestens mittelbar mit den Erfassungsmitteln (112) verbundenen Wamsignalgeber (113), der insbesondere über eine Steuereinheit (101) mit dem Ausgang der Vergleichereinheit (111) verbunden ist.

9. Implantierbares Stimulationsgerät nach einem der vorangehenden Ansprüche, **gekennzeichnet durch** die Ausbildung als Muskel- oder Nervenstimulator, insbesondere Herzschrittmacher (100), mit einer bidirektionalen Telemetrieeinrichtung (102) zur externen Programmierung der Stimulationsenergie und zur Übermittlung von den Erschöpfungszustand der Spannungsquelle (106) kennzeichnenden Daten nach extern im Rahmen einer Programmierung.

## Claims

1. An implantable stimulation device (100), having a depletable voltage source (106) for the energy supply, a control mechanism (101, 105) for controlling a stimulation operation, an output stage (107) having a stimulation energy accumulator (107a), means for generating an output voltage that is higher than the terminal voltage of the depletable voltage source by the supply of energy from the depletable voltage source into the stimulation energy storage device and a pumping control device (110, 111, 112; 110', 112a') for the time and/or current intensity control of the energy supply into the stimulation energy storage device as a function of the depletion state of the voltage source, wherein the pumping control device comprises means (112; 112a') for recording the state, in particular the terminal voltage, of the voltage source (106) and a controllable pumping current limiter circuit (110.9') that can be connected at least indirectly at the input side to the recording means and/or a pumping time interval control device (110.2', 110.5') for limiting the pumping current in connection with an extension of the pumping time interval as the depletion of the voltage source increases, so that a minimum operating voltage that is required for the safe operation of the control device is not fallen short of,
**characterised by** a first random-access memory (110.2'), connected via a first file server (110.1') to the output of the recording means (112, 112a'), for storing a plurality of values of the pumping time interval and a second random-access memory (110.7') that can be addressed via a second file server (110.6') for storing values of a control variable of the pumping current limiter circuit (110.9').

2. An implantable stimulation device according to Claim 1,
**characterised in that** the first random-access memory (110.2') is a programmable memory.

3. An implantable stimulation device according to Claim 1 or Claim 2,
**characterised in that** the second random-access memory (110.7') is a programmable memory.

4. An implantable stimulation device according to one of Claims 1 to 3,
**characterised in that** the pumping time-interval control mechanism (110.2', 110.5') is configured for the phased extension of the pumping time interval within the limits of the time interval between two stimulation pulses in response to a signal of the recording means (112; 112a') that marks an increasing depletion of the voltage source (106).

5. An implantable stimulation device according to one of the preceding Claims,
**characterised by** a comparator unit (111) that is connected via a first input to the recording means (112) and is connected via a second input to a read-only memory (104a) for storing the minimum operating voltage and also at the output side to a control input of the pumping current limiter circuit (110).

6. An implantable stimulation device according to Claim 5,
**characterised in that** the read-only memory (104a) is configured to store several graduated operating voltage threshold values and the comparator unit (111) is configured as a multi-stage threshold value discriminator in such a manner that the comparator unit emits different output signals as a function of the falling short in each case of one of the determined stored operating voltage threshold values at the battery voltage transducer (112).

7. An implantable stimulation device according to one of Claims 1 to 6,
**characterised in that** the pumping current limiter circuit (110.9') comprises a peak-current limiter, in particular a multi-stage peak-current limiter.

8. An implantable stimulation device according to one of Claims 1 to 7,
**characterised by** a warning signal generator (113) connected at least indirectly to the recording means (112), which is connected to the output of the comparator unit (111) in particular via a control unit (101).

9. An implantable stimulation device according to one of the preceding Claims,
**characterised by** the configuration as a muscle or nerve stimulator, in particular a cardiac pacemaker (100), having a bidirectional telemetry device (102) for the external programming of the stimulation energy and for transferring data that characterises the depletion state of the voltage source (106) to the outside within the scope of a programming operation.

## Revendications

1. Stimulateur implantable (100) comportant une source de tension (106) pouvant s'épuiser et servant à réaliser l'alimentation en énergie, un dispositif de commande (101, 105) pour commander le fonctionnement de stimulation, un étage de sortie (107) comportant un accumulateur d'énergie de stimulation (107a), des moyens pour produire une tension de sortie accrue par rapport à la tension aux bornes de la source de tension pouvant s'épuiser, par apport d'énergie à partir de la source de tension pouvant s'épuiser, dans l'accumulateur d'énergie de stimulation et un dispositif de commande de pompe (110, 111, 112; 110', 112a') pour réaliser la commande temporelle et/ou la commande de l'intensité du courant de l'apport d'énergie dans l'accumulateur d'énergie de stimulation en fonction de l'état d'épuisement de la source de tension, et dans lequel le dispositif de commande de pompe comprend des moyens (112; 112a') pour détecter l'état, notamment la tension aux bornes, de la source de tension (106) et un circuit commandable (110.9') de limitation du courant de la pompe et/ou un dispositif commandable (110.2', 110.5') de commande de l'intervalle de pompage, qui est relié au moins indirectement côté entrée aux moyens de détection, pour limiter le courant de la pompe en liaison avec un allongement de l'intervalle de pompage lorsque l'appauvrissement de la source de tension augmente, de sorte que la tension de fonctionnement ne tombe pas au-dessous de la tension de fonctionnement minimale nécessaire pour un fonctionnement sûr du dispositif de commande, **caractérisé par** une première mémoire à accès direct (110.2'), reliée par l'intermédiaire d'une première unité de commande d'accès en mémoire (110.1') à la sortie des moyens de détection (112, 112a'), pour la mémorisation d'une multiplicité de valeurs de l'intervalle de pompage et par une seconde mémoire à accès direct (110.7'), qui peut être adressée par l'intermédiaire d'une seconde commande d'accès en mémoire (110.6'), pour la mémorisation de valeurs d'une grandeur de commande du circuit de limitation (110.9') du courant de pompage.

2. Stimulateur implantable selon la revendication 1, **caractérisé en ce que** la première mémoire à accès direct (110.2') est une mémoire programmable.

3. Stimulateur implantable selon la revendication 1 ou la revendication 2, **caractérisé en ce que** la seconde mémoire à accès direct (110.7') est une mémoire programmable.

4. Stimulateur implantable selon l'une des revendications 1 à 3, **caractérisé en ce que** le dispositif (110.2', 110.5') de commande des intervalles de pompage est conçu pour l'allongement échelonné de l'intervalle de temps de pompage dans les limites de l'intervalle de temps entre deux impulsions de stimulation en réponse à un signal des moyens de détection (112; 112a'), qui caractérise un épuisement croissant de la source de tension (106).

5. Stimulateur implantable selon l'une des revendications précédentes, **caractérisé par** une unité formant comparateur (111) reliée par une première entrée aux moyens de détection (112) et par une seconde entrée à une mémoire morte (104a) servant à mémoriser la tension de fonctionnement minimale, ainsi que, côté sortie, à une entrée de commande du circuit (110) de limitation du courant de pompage.

6. Stimulateur implantable selon la revendication 5, **caractérisé en ce que** la mémoire morte (104a) est agencée de manière à mémoriser plusieurs valeurs de seuil échelonnées de la tension de fonctionnement et que l'unité formant comparateur (111) est agencée sous la forme d'un discriminateur à valeur de seuil à plusieurs étages de telle sorte que l'unité formant comparateur délivre différents signaux de sortie en fonction du dépassement, par le bas, respectivement d'une valeur déterminée parmi les valeurs de seuil mémorisées de la tension de fonctionnement dans le capteur (102) de la tension de la pile.

7. Stimulateur implantable selon l'une des revendications 1 à 6, **caractérisé en ce que** le circuit (110.9') de limitation du courant de pompage possède un limiteur du courant maximum, notamment à plusieurs échelons.

8. Stimulateur implantable selon l'une des revendications 1 à 7, **caractérisé par** un transmetteur de signaux d'avertissement (113), qui est relié au moins indirectement aux moyens de détection (112) et est relié notamment au moyen de l'unité de commande (101) à la sortie de l'unité formant comparateur (111).

9. Stimulateur implantable selon l'une des revendications précédentes, **caractérisé par** son agencement sous la forme d'un stimulateur musculaire ou nerveux, notamment d'un stimulateur cardiaque (100), comportant un dispositif de télémétrie bidirectionnel (102) pour programmer de façon externe l'énergie de stimulation et pour réaliser la transmission, vers l'extérieur, de données caractérisant l'état d'épuisement de la source de tension (106) dans le cadre d'une programmation.
